# EUROPEAN PATENT SPECIFICATION

(11) **EP 2 068 946 B1**
(45) Date of publication and mention of the grant of the patent: **15.08.2012**
(21) Application number: 07808585.9
(22) Date of filing: 18.09.2007
(51) Int. Cl.: A61L 9/12, B60H 3/00

(54) **DUAL SCENT AIR-FRESHENING DISPENSING DEVICE**
LUFTAUFFRISCHER-SPENDEVORRICHTUNG MIT ZWEIFACHEM DUFT
DISPOSITIF DÉODORISANT POUR DISTRIBUER DEUX ODEURS

(30) Priority: 19.09.2006 EP 06076744
(43) Date of publication of application: 17.06.2009
(73) Proprietor: The Procter & Gamble Company, Cincinnati, OH 45202 (US)
(72) Inventor: BULSINK, Dirk, Jan, NL-2331 BZ Leiden (NL); BOGERD, Caspar, Barry, NL-2761 GA Zevenhuizen (NL)
(74) Representative: Morelle, Evelyne Charlotte Isabelle
(86) International application number: PCT/NL2007/050454
(87) International publication number: WO 2008/035967

(56) References cited:
- EP-A- 1 541 396
- EP-A2- 1 486 365
- WO-A1-03/028775
- GB-A- 2 401 047
- US-A- 4 523 870
- US-A1- 2004 247 301

## Description

The invention relates to an air-freshening dispensing device. In particular, it relates to a dispensing device for delivering a fragrance, for example a deodorising agent or a fragrance, for instance in an automobile.

WO2003/028775 discloses a dispensing unit for dispensing a combination of fragrances, wherein a pair of mounts is provided for mounting respective fragrance containers in outer sections of the device. A switchable shutter element is arranged for selectively shutting access of the airflow to any of the outer sections. The device is dependent on a reversal of air stream flow of a fan to provide any of a selected fragrance. This mechanism is not practical for applications which are used in conjunction with an external fan, for instance, in automobiles.

EP200476393 discloses an air-freshening dispensing device for a vehicle. The device can be clamped onto an outlet channel of a car fan and comprises a mount for mounting a fragrance container. The device only comprises a single fragrance container.

GB2401047A relates to a fragrance dispenser including a first source of fragrance associated with a first flow path and a second source of fragrance associated with a second flow path. A fan is provided for providing an flow of air along each flowpath. A shutter is moveable by an actuator rod to open and close the flowpaths so that only one fragrance is released at a time to dispense a proportionate mixtures of the two fragrances.

EP1486365A2 discloses a device for diffusing volatile substances, particularly deodorants for vehicle interiors, comprising a container designed to contain a liquid volatile substance to be diffused, a wick disposed partially in the container and a lid applied to the container around one end of the wick.

It is desirable to provide a duo fragrance-dispensing device which is compact and provides a possibility to selectable fragrance.

In accordance with an aspect of the present invention there is provided an air-freshening dispensing device according to the features of claim 1. In particular, there is provided an air-freshening dispensing device, comprising: a housing, comprising a pair of mounts for mounting respective containers provided in outer sections of said housing; and a switchable shutter element arranged for selectively shutting access of an airflow to either of the outer sections; wherein said shutter element is provided in a mid section between said outer sections; said mid section being communicatively coupled to an air inlet, and further comprising an air outlet for providing throughput of the airflow through the mid section; the device further comprising a clamp for clamping the unit onto an air outlet channel to provide an airflow into the air inlet.

A device thus configured may provide compact and selective switching, wherein, when in use, only one fragrance is selected and the other is shut off from the airflow. Accordingly, a cost effective and efficient device for providing multiple fragrances can be provided.

Further features and benefits can be derived from the description, read in conjunction with the figures. In the figures:
Figure 1 shows a schematic perspective view that is partly shown in cross sectional view, of a dispenser according to an aspect of the invention;
Figures 2A and B show alternative working conditions wherein a shutter element alternatively provides and shuts access to an evaporation surface of a fragrance;
Figure 3 shows a schematic perspective cross sectional view of the dispensing device of Figure 1 sectioned along line I-I, showing the shutter in a selected shutting position; and
Figure 4 shows a top view of the slider through a top cross sectional view of the dispenser of Figure 1 sectioned along line II-II.

In the figures, the same or corresponding items are referenced by the same reference numerals.

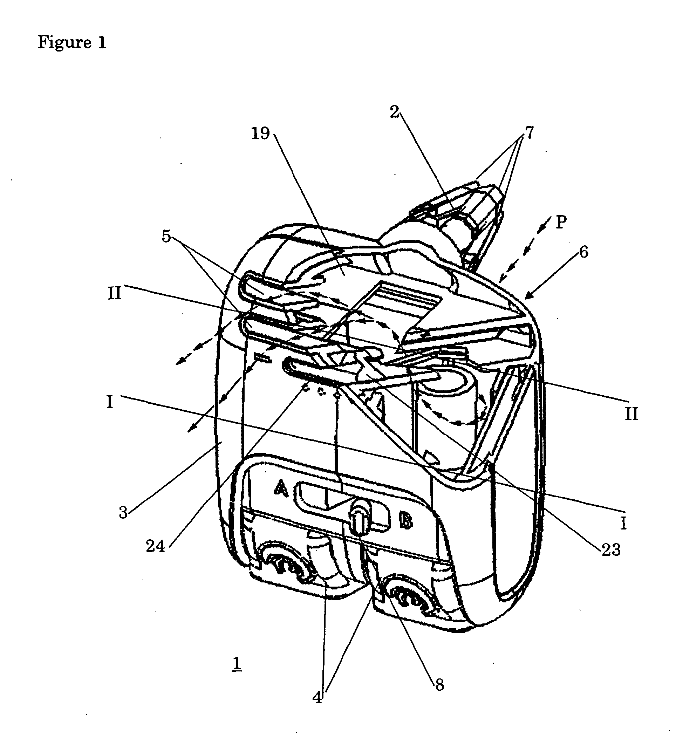

In Figure 1 a front perspective schematic view of an embodiment of the dispensing device or air freshener 1 according to the invention is illustrated. The air-freshener 1 is attachable to a site in the interior of a vehicle, in particular a motorcar, in a conventional way, for example, by the clamping mechanism 2 explained below. The air-freshener 1 is provided with a housing 3 into which containers 4 for a volatile liquid can be inserted (indicated by references A and B in the figure). The front comprises ventilation outlets 5 via which air can flow through the air freshener 1.

The rear comprises ventilation openings 6, that allow air to be transported into the housing 3. A clamping element 2 is present for securing the air-freshener 1 on a strip-shaped element, in particular that of a guard of the fan in the dashboard of a motorcar. The clamping element 2 comprises four clamps 7 projecting outwards from the housing 3, which clamps 7 are separated from each other according to two planes which are at least substantially perpendicular to each other, to enable securing the air-freshener 1 either on a vertical, or on a horizontal strip-shaped element.

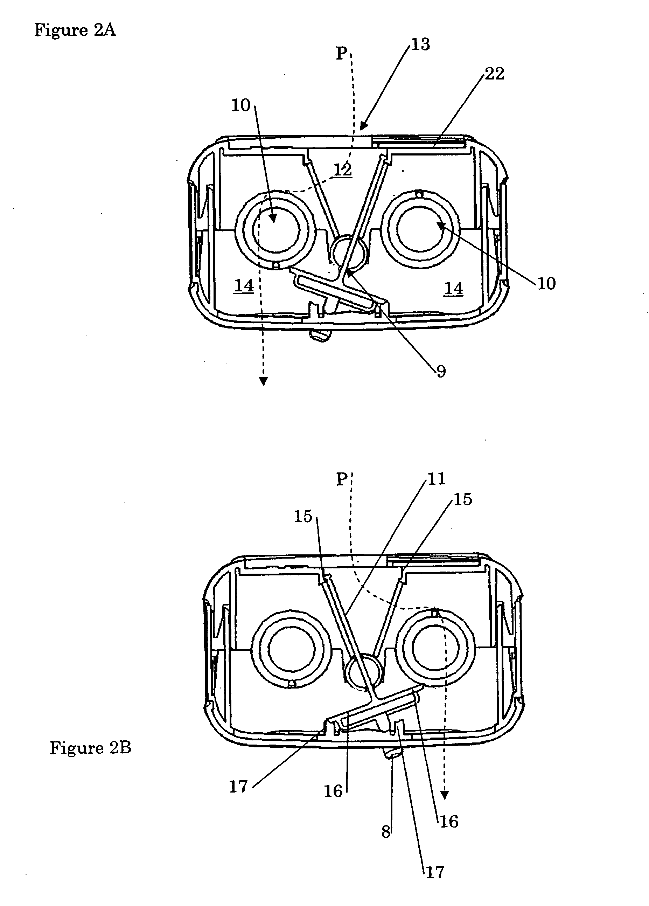

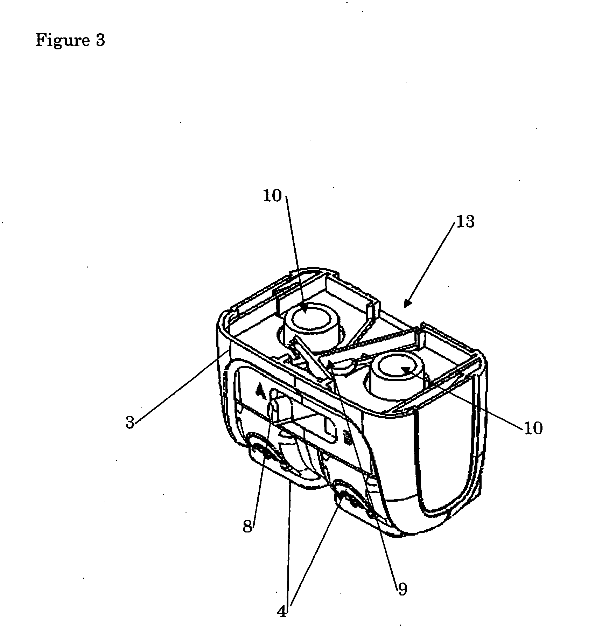

Figure 1 further shows schematically the path of the airflow indicated by arrows P. A switchable shutter element, further clarified and referenced with numeral 9 in subsequent figures, is arranged for selectively shutting access of the airflow P to any outer section of the housing 3 wherein containers 4 are provided. The shutter element is provided in a mid section between said outer sections and comprises a knob 8 protruding through the housing 3 to switch a valve piece (see Figure 2) to any of the opposite sides of the mid section, as will be more clearly illustrated in Figure 2 and Figure 3.

In particular, Figures 2A and B show alternative working conditions wherein a shutter element 9 alternatively provides and shuts access to a fragrance provided via an evaporation surface in the form of a wick 10 that protrudes from containers 4 which comprise mutually different fragrances. Thus, the Figure 2A configuration provides access to the left hand wick 10, viewed from above, while shutting access to the right hand wick 10; and the Figure 2B configuration provides access to the right hand wick 10, viewed from above, while shutting access to the left hand wick 10. Furthermore, Figure 3 shows a schematic perspective cross sectional view of the dispensing device of Figure 1 sectioned along line I-I, showing the shutter in a selected shutting position.

Although in principle, via this mechanism, a mixture of fragrances can be provided by selectively opening or shutting access to any of the left and right hand wicks 10, preferably, the shutter element 9 is bistably switchable, so that only a single fragrance is activated by switching the knob 8 either to the "A" or the "B"- state, to select either a fragrance from the left hand or the right hand container. In particular, Figures 2 and 3 show that preferably, the shutter element 9 comprises a valve piece 11 that is pivotably mounted in mid section 12, to include or exclude an air inlet 13 to either of outer sections 14, by pivoting the valve piece 11 against a seat structure 15 provided in the housing 3.

Furthermore, Figure 2 and Figure 3 show that the seat structure 15 is preferably wedge shaped, so that the valve piece 11 seats against either of opposite sides of the wedge shaped seat structure 15. In this wedge shape form, preferably, the valve piece comprises cross-shaped flaps 16, oriented transversely to said valve piece 11. The flaps 16, by pivoting the valve piece 11, open or close access to any of the outer sections 14 through abutting a side of a flap against a seat 17 arranged in the outer section 14.

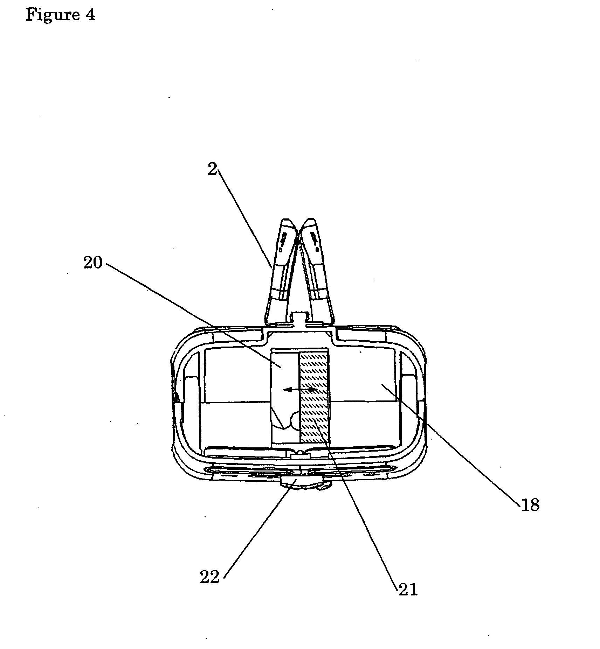

Figure 4 shows a top view of a slider 21 through a top cross sectional view of the dispenser of Figure 1 sectioned along line II-II. In particular, a lower wall 18 of a collection chamber 19 is illustrated (see also Figure 1). As can be seen in Figure 1, the collection chamber 19 is provided with an air outlet 5. Accordingly, access to this collection chamber 19 is provided via a central opening 20 that is slidably closable through slider 21. To prevent airflow near the wick 10 in a closed condition by the slider 21, additionally, preferably, the slider element 21 also slidingly closes the air inlet 13. Thus, preferably, the slider 21 comprises another closure element (not shown) slidable along an inlet opening 13 provided in a first side wall, typically, the back wall 22 (see Figure 2) of the mid section 12 forming the air inlet 13, for slidingly closing the air inlet 13 access to said mid section. This closure element is provided in addition to the closure element 21 slidable along the air outlet opening provided in a top wall 18 of the mid section 12, which is at the same time the bottom wall 18 of the collection chamber 19. Although the embodiment illustrates a slider 21 that slidingly closes the openings in a top wall (reference by 20) and a sidewall of the mid section 12 (not shown), also other walls can be slidingly shutted, for example, opposite walls, according to this principle. The slider is manually operable via a slider knob 23 that is slidable along a slit 24 provided in the housing 3 (see Figure 1).

## Claims

1. An air-freshening dispensing device (1), attachable to a site in the interior of a vehicle by a clamping mechanism (2) comprising:
- a housing, (3) comprising a pair of device, comprising: respective containers (4) provided in outer sections of said housing (3); and
- a switchable shutter element (9) arranged for selectively shutting access of
an airflow to any of the outer sections;
- said shutter element (9) is provided in a mid section (12) between said outer
sections (14);
- said mid section (12) being communicatively coupled to an air inlet (13), and further comprising an air outlet (5); for providing throughput of the airflow through the mid section (12)
**characterized in that** the shutter element (9) comprises a valve piece (11) that is mounted in the mid section (12), to include or exclude the air inlet ta either of the outer sections, by pivoting the valve piece (11) against a seat structure (15) provided in the housing (3), and the mid section (12) is bounded by a wedge shaped seat structure (15), the valve piece (11) seating against either of opposite sides of the wedge shaped seat structure (15); and further comprising a knob (8) protruding through the housing (3) to switch the valve to any of the opposite sides of the mid section (12).

2. A dispensing device (1) according to claim 1, further comprising a clamp (2) for clamping the device (1) onto an outlet channel to provide an airflow into the air inlet.

3. A dispensing device (1) according to claim 1, wherein the shutter element (9) is bistably switchable.

4. A dispensing device (1) according to claim 1 wherein the valve piece (11) further comprises cross-shaped flaps (16), oriented transversely to said valve piece (11).

5. A dispensing device (1) according to claim 4, wherein the flaps (16), by pivoting the valve piece (11), open or close access to any of the outer sections (14) through abutting a side of a flap (16) against a seat arranged in the outer section (14).

6. The dispensing device (1) of claim 1, further comprising a slider element (21) that slidingly shuts the air inlet, for regulating an airflow intensity.

7. The dispensing device (1) of claim 6, wherein the slider (21) comprises a closure element slidable along an inlet opening provided in a first side wall of the mid section, for slidingly closing the air inlet access to said mid section (12), and wherein the slider further comprises a second closure element slidable along an air outlet opening provided in a second side wall of the mid section (12).

8. The dispensing device (1) of claim 7, wherein said first and second side walls are adjacent; and wherein said second side wall is a top wall when viewed in mounted condition.

9. The dispensing device (1) of claim 7, wherein said first and second side walls are opposite to each other.

10. The dispensing device of claim 8, wherein said top wall defines a lower wall of a collection chamber (19) that is provided with an air outlet (5).

## Patentansprüche

1. Lufterfrischer-Abgabevorrichtung (1), die durch einen Klemmmechanismus (2) an einer Stelle im Inneren eines Fahrzeugs befestigt werden kann und Folgendes umfasst:
- ein Gehäuse (3), das ein Paar Halterungen zum Befestigen entsprechender Behälter (4), die in Außenabschnitten des Gehäuses (3) bereitgestellt sind, umfasst, und
- ein umstellbares Klappenelement (9), das so angeordnet ist, dass auf Wunsch ein Luftstrom zu einem der Außenabschnitte unterbrochen werden kann,
- wobei das Klappenelement (9) in einem Mittelabschnitt (12) zwischen den Außenabschnitten (14) bereitgestellt ist,
- wobei der Mittelabschnitt (12) kommunikativ mit einem Lufteinlass (13) gekoppelt ist und ferner einen Luftauslass (5) umfasst, um den Durchsatz des Luftstroms durch den Mittelabschnitt (12) zu ermöglichen,
**dadurch gekennzeichnet, dass** das Klappenelement (9) ein Ventilstück (11) umfasst, das im Mittelabschnitt (12) befestigt ist, um den Lufteinlass in einen der beiden Außenabschnitte hinein bzw. hinaus zu ermöglichen bzw. zu blockieren, indem das Ventilstück (11) gegen eine Sitzstruktur (15) im Gehäuse (3) geschwenkt wird, und wobei der Mittelabschnitt (12) durch eine keilförmige Sitzstruktur (15) begrenzt wird, wobei das Ventilstück (11) gegen eine der entgegengesetzten Seiten der keilförmigen Sitzstruktur (15) anschlägt, und wobei die Lufterfrischer-Abgabevorrichtung (1) ferner einen Knopf (8) umfasst, der durch das Gehäuse (3) hindurchragt, um das Ventil auf eine der entgegengesetzten Seiten des Mittelabschnitts (12) umzustellen.

2. Abgabevorrichtung (1) nach Anspruch 1, die ferner eine Klemme (2) zum Festklemmen der Vorrichtung (1) auf einen Auslasskanal zum Bereitstellen eines Luftstroms in den Lufteinlass umfasst.

3. Abgabevorrichtung (1) nach Anspruch 1, wobei das Klappenelement (9) ein Kippschalter ist.

4. Abgabevorrichtung (1) nach Anspruch 1, worin das Ventilstück (11) ferner kreuzförmige Klappen (16) umfasst, die quer zum Ventilstück (11) ausgerichtet sind.

5. Abgabevorrichtung (1) nach Anspruch 4, wobei die Klappen (16) durch das Schwenken des Ventilstücks (11) den Zugang zu einem der Außenabschnitte (14) ermöglichen bzw. blockieren, indem eine Seite einer Klappe (16) gegen einen Sitz im Außenabschnitt (14) anstößt.

6. Abgabevorrichtung (1) nach Anspruch 1, die ferner ein Schiebeelement (21) umfasst, mit dem durch ein Verschieben der Lufteinlass abgesperrt werden kann, zum Steuern der Luftstromintensität.

7. Abgabevorrichtung (1) von Anspruch 6, wobei der Schieber (21) ein Verschlusselement umfasst, das entlang einer Einlassöffnung in einer ersten Seitenwand des Mittelabschnitts verschoben werden kann, um den Luftstrom durch den Lufteinlass hin zum Mittelabschnitt (12) durch ein Verschieben abzusperren, und wobei der Schieber ferner ein zweites Verschlusselement umfasst, das entlang einer Luftauslassöffnung in einer zweiten Seitenwand des Mittelabschnitts (12) verschoben werden kann.

8. Abgabevorrichtung (1) nach Anspruch 7, wobei die erste und zweite Seitenwand aneinander angrenzen, und wobei die zweite Seitenwand bei Betrachtung in befestigtem Zustand eine obere Wand ist.

9. Abgabevorrichtung (1) nach Anspruch 7, wobei die erste und zweite Seitenwand einander gegenüber liegen.

10. Abgabevorrichtung nach Anspruch 8, wobei die obere Wand eine untere Wand einer Auffangkammer (19) bildet, die einen Luftauslass (5) aufweist.

## Revendications

1. Dispositif dispensateur de désodorisant (1) pouvant être fixé à un site à l'intérieur d'un véhicule par un mécanisme de serrage (2), comprenant :
- un logement (3), comprenant une paire de montures pour monter des récipients respectifs (4) fournis dans des sections externes dudit logement (3) ; et
- un élément obturateur commutable (9) arrangé pour obturer de façon sélective l'accès d'un flux d'air à n'importe laquelle des sections externes ;
- ledit élément obturateur (9) est fourni dans une section intermédiaire (12) entre lesdites sections externes (14) ;
- ladite section intermédiaire (12) étant couplée par communication avec une entrée d'air (13), et comprenant en outre une sortie d'air (5) pour fournir un débit du flux d'air à travers la section intermédiaire (12) ;
- **caractérisé en ce que** l'élément obturateur (9) comprend une pièce de valve (11) qui est montée dans la section intermédiaire (12), pour inclure ou exclure l'entrée d'air vers l'une ou l'autre des sections externes, en pivotant la pièce de valve (11) contre une structure d'appui (15) fournie dans le logement (3), et la section intermédiaire (12) est délimitée par une structure d'appui biseautée (15), la pièce de valve (11) s'appuyant contre l'un ou l'autre des côtés opposés de la structure d'appui biseautée (15) ; et comprenant en outre un bouton (8) faisant saillie à travers le logement (3) pour mettre la valve sur n'importe lequel des côtés opposés de la section intermédiaire (12).

2. Dispositif dispensateur (1) selon la revendication 1, comprenant en outre une pince (2) pour serrer le dispositif (1) sur un canal de sortie pour fournir un flux d'air dans l'entrée d'air.

3. Dispositif dispensateur (1) selon la revendication 1, dans lequel l'élément obturateur (9) est commutable de façon bistable.

4. Dispositif dispensateur (1) selon la revendication 1, dans lequel la pièce de valve (11) comprend en outre des volets en forme de croix (16), orientés transversalement sur ladite pièce de valve (11).

5. Dispositif dispensateur (1) selon la revendication 4, dans lequel les volets (16), en pivotant la pièce de valve (11), ouvrent ou ferment l'accès à l'une quelconque des sections externes (14) en amenant en butée un côté d'un volet (16) contre une surface d'appui arrangée dans la section externe (14).

6. Dispositif dispensateur (1) selon la revendication 1, comprenant en outre un élément de glissière (21) qui obture de façon coulissante l'entrée d'air, pour réguler l'intensité du flux d'air.

7. Dispositif dispensateur (1) selon la revendication 6, dans lequel la glissière (21) comprend un élément de fermeture pouvant coulisser le long d'une ouverture d'entrée constituée dans une première paroi latérale de la section intermédiaire, pour fermer de façon coulissante l'accès d'entrée d'air à ladite section intermédiaire (12), et dans lequel la glissière comprend en outre un deuxième élément de fermeture pouvant glisser le long d'une ouverture de sortie d'air constituée dans une deuxième paroi latérale de la section intermédiaire (12).

8. Dispositif dispensateur (1) selon la revendication 7, dans lequel lesdites première et deuxième parois latérales sont adjacentes ; et dans lequel ladite deuxième paroi latérale est une paroi supérieure lorsqu'on observe dans un état monté.

9. Dispositif dispensateur (1) selon la revendication 7, dans lequel lesdites première et deuxième parois latérales sont opposées l'une à l'autre.

10. Dispositif dispensateur selon la revendication 8, dans lequel ladite paroi supérieure définit une paroi inférieure d'une chambre de recueil (19) qui est pourvue d'une sortie d'air (5).
